(19) Europäisches Patentamt
European Patent Office
Office européen des brevets

(11) Veröffentlichungsnummer: **0 135 084**
**A2**

(12) # EUROPÄISCHE PATENTANMELDUNG

(21) Anmeldenummer: **84109026.9**

(22) Anmeldetag: **31.07.84**

(51) Int. Cl.⁴: **C 07 D 217/04**
**A 01 N 47/18**

(30) Priorität: **11.08.83 DE 3329098**

(43) Veröffentlichungstag der Anmeldung: **27.03.85 Patentblatt 85/13**

(84) Benannte Vertragsstaaten: **CH DE FR GB IT LI**

(71) Anmelder: **BAYER AG**
**Konzernverwaltung RP Patentabteilung**
**D-5090 Leverkusen 1 Bayerwerk(DE)**

(72) Erfinder: **Ippen, Joachim, Dr.**
**Morgengraben 14**
**D-5000 Koeln 80(DE)**

(72) Erfinder: **Heywang, Gerhard, Dr.**
**Nittumer Weg 5**
**D-5060 Bergisch-Gladbach 2(DE)**

(72) Erfinder: **Becker, Benedikt, Dr.**
**Metzkausenerstrasse 14**
**D-4020 Mettmann(DE)**

(72) Erfinder: **Hammann, Ingeborg, Dr.**
**Lutherstrasse 22**
**D-4330 Muelheim/Ruhr(DE)**

(72) Erfinder: **Homeyer, Bernhard, Dr.**
**Obere Strasse 28**
**D-5090 Leverkusen 3(DE)**

(54) **Neue Tetrahydroisochinolinyl-carbamidsäureester, Verfahren zu ihrer Herstellung und ihre Verwendung zur Schädlingsbekämpfung.**

(57) Die vorliegende Erfindung betrifft neue Tetrahydroisochinolinyl-carbamidsäureester der Formel (I)

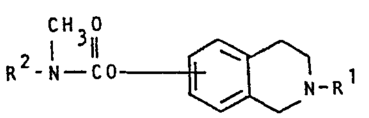 (I)

in welcher

$R^1$ für gegebenenfalls substituiertes Alkyl, Alkenyl oder Alkinyl steht,

$R^2$ für Wasserstoff, Alkyl, oder den Rest $R^3$-S- steht, wobei $R^3$ für gegebenenfalls halogensubstituiertes Alkyl, oder gegebenenfalls substituiertes Dialkylamino steht, wobei die beiden Alkylreste am Stickstoffatom gegebenenfalls über ein Heteroatom (Sauerstoff, gegebenenfalls alkylsubstituierter Stickstoff oder Schwefel) einen Ring bilden können, oder für acyliertes Alkylamino steht.

Sie werden erhalten, indem man Hydroxy-tetrahydroisochinoline der Formel (II)

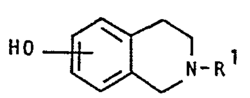 (II)

a) mit Methylisocyanat umsetzt oder

b) mit Carbamidsäurehalogeniden der Formel (III)

$CH_3$
|
$R^2-N-CO\ X$ (III)

umsetzt, oder

c) Verbindungen der Formel (IV)

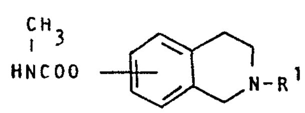 (IV)

mit Sulfensäurechloriden der Formel (V)

$R^3-S-Cl$ (V)

umsetzt. Sie eignen sich zur Bekämpfung von Schädlingen.

EP 0 135 084 A2

Croydon Printing Company Ltd

BAYER AKTIENGESELLSCHAFT 5090 Leverkusen, Bayerwerk

Konzernverwaltung RP
Patentabteilung

Rt/by-c
Ia

Neue Tetrahydroisochinolinyl-carbamidsäureester, Verfahren zu ihrer Herstellung und ihre Verwendung zur Schädlingsbekämpfung

Die vorliegende Erfindung betrifft neue Tetrahydroisochinolinyl-carbamidsäureester, Verfahren zu ihrer Herstellung und ihre Verwendung zur Schädlingsbekämpfung.

Es ist bereits bekannt geworden, daß N-Methylcarbamidsäureester von Hydroxytetrahydrochinolinen insektizide Eigenschaften haben. Ihre Wirkung ist jedoch, vor allem bei niedrigen Aufwandmengen, nicht immer voll befriedigend (vgl. DE-OS 2 144 976).

Es wurden nun neue Tetrahydroisochinolinyl-carbamidsäureester der Formel (I) gefunden

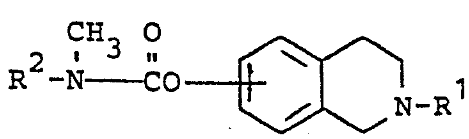

(I)

in welcher

$R^1$ für gegebenenfalls substituiertes Alkyl, Alkenyl oder Alkinyl steht,

$R^2$ für Wasserstoff, Alkyl oder den Rest $R^3$-S- steht, wobei $R^3$ für gegebenenfalls halogensubstituiertes Alkyl, oder gegebenenfalls substituiertes Dialkylamino steht, wobei die beiden Alkylreste am Stickstoffatom gegebenenfalls über ein Heteroatom (Sauerstoff, gegebenenfalls alkylsubstituierter Stickstoff oder Schwefel) einen Ring bilden können, oder für acyliertes Alkylamino steht.

Weiterhin wurde gefunden, daß man die neuen Tetrahydroisochinolinyl-carbamidsäureester der Formel (I)

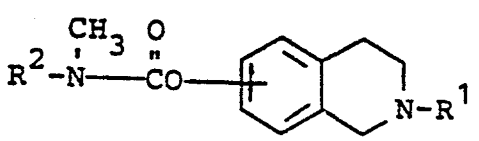

(I)

in welcher

$R^1$ für gegebenenfalls substituiertes Alkyl, Alkenyl oder Alkinyl steht,

$R^2$ für Wasserstoff, Alkyl oder den Rest $R^3$-S- steht, wobei $R^3$ für gegebenenfalls halogensubstituiertes Alkyl, oder gegebenenfalls substituiertes Dialkylamino

steht, wobei die beiden Alkylreste am Stickstoffatom gegebenenfalls über ein Heteroatom (Sauerstoff, gegebenenfalls alkylsubstituierter Stickstoff oder Schwefel) einen Ring bilden können, oder für acyliertes Alkylamino steht,

erhält, indem man Hydroxy-tetrahydroisochinoline der Formel (II)

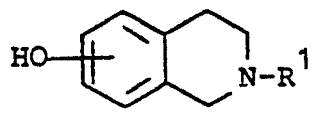 (II)

in welcher

$R^1$ die oben angegebene Bedeutung hat

a) in üblicher Weise mit Methylisocyanat umsetzt oder

b) mit Carbamidsäurehalogeniden der Formel (III)

$$R^2\text{-}\overset{CH_3}{\overset{|}{N}}\text{-CO-X} \qquad \text{(III)}$$

in welcher

$R^2$ die oben angegebene Bedeutung hat und

X für Halogen steht

gegebenenfalls in Gegenwart eines Säurebindemittels umsetzt, oder

c) Verbindungen der Formel (IV)

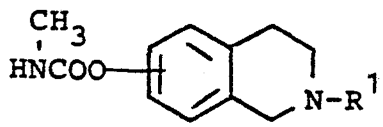

(IV)

in welcher

$R^1$ die oben angegebene Bedeutung hat,

mit Sulfensäurechloriden der Formel (V)

$$R^3\text{-S-Cl} \qquad (V)$$

in welcher

$R^3$ die oben angegebene Bedeutung hat,

gegebenenfalls in Gegenwart eines Säurebindemittels gegebenenfalls in Gegenwart von Katalysatoren umsetzt.

Weiterhin wurde gefunden, daß die Verbindungen der Formel (I) zur Bekämpfung von Schädlingen, vor allem von Arthropoden, vorzugsweise von Insekten und Spinnentieren, insbesondere von Insekten, verwendet werden können.

Es ist ausgesprochen überraschend, daß die erfindungsgemäßen Verbindungen der Formel (I) günstigere Eigenschaften aufweisen, als die vom Stand der Technik her bekannten N-Methylcarbamidsäureester von Hydroxytetrahydrochinolinen. Die erfindungsgemäßen Stoffe stellen somit eine Bereicherung der Technik dar.

Durch die Formel (I) sind die neuen Verbindungen allgemein definiert.

Bevorzugt sind Verbindungen der Formel (I), in welcher

$R^1$ für gegebenenfalls substituiertes Alkyl mit 1 bis 6 C-Atomen, für gegebenenfalls substituiertes Alkenyl mit 3 bis 6 C-Atomen oder für gegebenenfalls substituiertes Alkinyl mit 3 bis 6 C-Atomen und

$R^2$ für Wasserstoff, Alkyl mit 1 bis 4 C-Atomen, sowie den Rest $-S-R^3$ steht, $R^3$ steht für gegebenenfalls halogeniertes Alkyl mit 1 bis 3 C-Atomen, gegebenenfalls substituiertes Dialkylamino, wobei die beiden Alkylreste am Stickstoffatom auch gegebenenfalls über ein Heteroatom (Sauerstoff, gegebenenfalls durch $C_{1-4}$ substituierten Stickstoff, Schwefel) einen Ring bilden können, mit insgesamt 2 bis 16 C-Atomen stehen, oder für $C_{1-4}$-Alkoxycarbonyl-$C_{1-4}$-alkylamino.

Als mögliche Substituenten kommen Halogen, Cyan, Nitro, Alkyl mit 1 bis 2 C-Atomen, Alkyloxycarbonyl mit 1 bis 5 C-Atomen und Trifluormethyl in Betracht.

Der Carbamidsäurerest steht bevorzugt in 5- oder 7-Stellung.

Besonders bevorzugt sind Verbindungen der Formel (I), in welcher

$R^1$ für Methyl, Ethyl, Propyl, Butyl oder deren Isomeren, sowie für Allyl und Propargyl und

$R^2$ für Wasserstoff, Methyl, oder $-S-R^3$ steht, $R^3$ steht für Trichlormethyl, Fluordichlormethyl, Dimethylamino, Diethylamino, Dipropylamino, Dibutylamino, Dipentylamino, Di(ethoxycarbonylmethyl)amino, Pyrrolidino, Piperidino, Morpholino, N'-Methylpiperazino, Methoxycarbonyl-butylamino,

und in denen der Carbamidsäurerest in 5- oder 7-Stellung steht.

0135084

Besonders genannt seien die folgenden Verbindungen:

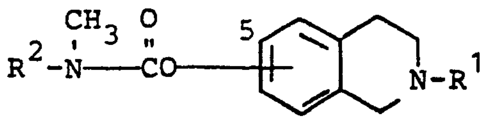

7

| $R^1$ | $R^2$ | Stellung des Carbamidsäurerestes |
|---|---|---|
| $CH_3$ | H | 5 |
| $CH_3$ | $CH_3$ | 5 |
| $CH_3$ | $-S-CCl_3$ | 5 |
| $CH_3$ | $-S-CCl_2F$ | 5 |
| $CH_3$ | $-S-N(C_4H_9t)$ | 5 |
| $CH_3$ | $-S-N(C_3H_7i)C_2H_4-COOC_2H_5$ | 5 |
| $CH_3$ | H | 7 |
| $CH_3$ | $CH_3$ | 7 |
| $CH_3$ | $-SCCl_3$ | 7 |
| $CH_3$ | $-SCCl_2F$ | 7 |
| $CH_3$ | $-S-N(C_4H_9t)$ | 7 |
| $CH_3$ | $-S-N(C_3H_7i)C_2H_4-COOC_2H_5$ | 7 |
| $-CH-C\equiv CH$ | H | 5 |
| $-CH-C\equiv CH$ | $-SCCl_3$ | 5 |
| $-CH-C\equiv CH$ | $-SCCl_2F$ | 5 |
| $-CH-C\equiv CH$ | -S-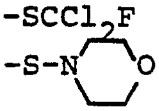 | 5 |
| $C_4H_9t$ | H | 7 |
| $C_4H_9t$ | $-S-CCl_3$ | 7 |
| $C_4H_9t$ | $-S-N(CH_3)_2$ | 7 |
| $C_4H_9t$ | $-S-N(C_3H_7i)C_2H_4-COOC_2H_5$ | 7 |

Verwendet man in Verfahren a) z.B. 5-Hydroxy-1,2,3,4-tetrahydroisochinolin und Methylisocyanat als Ausgangsstoffe, so läßt sich der Reaktionsverlauf durch das folgende Formelschema darstellen.

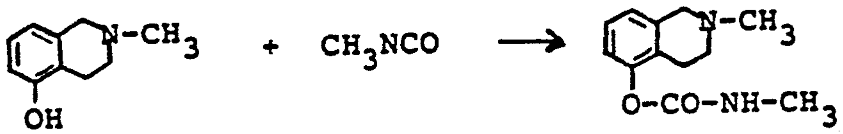

Die Verbindungen der Formel (II) sind bekannt und/oder können nach den in Bull. Chem. Soc. France 1961, 270 und Compt. Rend. 248, 426 (1959) angegebenen Verfahren hergestellt werden.

Verfahren (a) kann ohne Verdünnungsmittel oder in Gegenwart eines inerten Verdünnungsmittels durchgeführt werden. Als Verdünnungsmittel sind Kohlenwasserstoffe wie z.B. Hexan, chlorierte Kohlenwasserstoffe wie z.B. Methylenchlorid, aromatische Verbindungen wie z.B. Toluol, Nitrile wie z.B. Acetonitril, Ketone z.B. Aceton oder Gemische dieser Lösungsmittel geeignet.

Dem Reaktionsgemisch setzt man vorzugsweise einen Katalysator zu; geeignet sind besonders Triethylamin, Diazabicyclooctan und Dibutylzinndilaurat. Die Reaktionstemperatur kann in einem größeren Bereich variiert werden. Im allgemeinen arbeitet man zwischen 20 und 180°C. Besonders bevorzugt ist der Temperaturbereich von 40 bis 150°C.

Die Mengenverhältnisse der Ausgangsstoffe können im üblichen Rahmen variiert werden. Die jeweils angegebenen molaren Mengen müssen nicht genau eingehalten werden, sondern können nach oben oder unten abweichen.

Die Umsetzungen werden üblicherweise unter Normaldruck durchgeführt. Aber auch die Versuchsführung bei Über- oder Unterdruck kann Vorteile bieten.

Verwendet man im Verfahren b) z.B. 7-Hydroxy-2-methyl-1,2,3,4-tetrahydroxyisochinolin und N-(Dibutylaminosulfenyl)-N-methyl-carbamidsäurefluorid als Ausgangsstoffe, so läßt sich der Reaktionsverlauf durch das folgende Formelschema darstellen

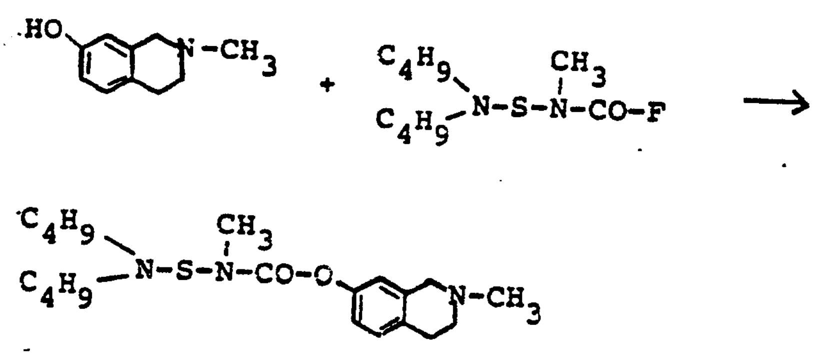

Die sulfenylierten Carbamidsäurehalogenide der Formel (III) sind bekannt und/oder können nach den in DE-AS 1 297 095, DE-OS 2 254 359, DE-OS 2 654 313, DE-OS 2 628 575 angegebenen Verfahren hergestellt werden.

Das Verfahren wird vorzugsweise in Gegenwart eines Verdünnungsmittels durchgeführt. Als Verdünnungsmittel sind Kohlenwasserstoffe, z.B. Hexan, chlorierte Kohlenwasserstoffe wie z.B. Methylenchlorid, aromatische Verbindungen wie z.B. Toluol, Nitrile wie z.B. Acetonitril, Ether wie z.B. Diethylether oder Tetrahydrofuran, aprotische dipolare Lösungsmittel wie Dimethylformamid, Dimethylsulfoxid und Hexamethylphosphorsäuretriamid oder Gemische dieser Verdünnungsmittel geeignet.

Dem Reaktionsgemisch setzt man vorzugsweise eine Base zu. Geeignet sind hier tertiäre organische Amine, wie Triethylamin, N,N-Dimethylanilin, N,N-Dimethylbenzylamin, Pyridin, Chinolin u.a. oder anorganische Basen wie Natriumhydrid, Natriumhydroxid, Kaliumcarbonat oder Natriumfluorid.

Die Reaktion kann in einem größeren Bereich variiert werden. Im allgemeinen arbeitet man zwischen -20° und 140°C. Besonders bevorzugt wird der Temperaturbereich von 0°C bis 100°C. Die Mengenverhältnisse der Ausgangsstoffe können im üblichen Rahmen variiert werden. Die Reihenfolge des Zusammengebens der Reaktionskomponenten ist beliebig. Die jeweils angegebenen molaren Mengen müssen nicht genau eingehalten werden; es können eine oder mehrere Komponenten im Überschuß eingesetzt werden.

Die Umsetzungen werden üblicherweise unter Normaldruck durchgeführt. Aber auch die Versuchsführung bei Über- oder Unterdruck kann Vorteile bieten.

Im Verfahren c) wird ein nach einem der obengenannten Verfahren a) oder b) erhältlicher N-Methyl-isochinolinylcarbamidsäureester mit Sulfensäurechloriden umgesetzt. Die Reaktion läßt sich durch folgendes Formelschema wiedergeben:

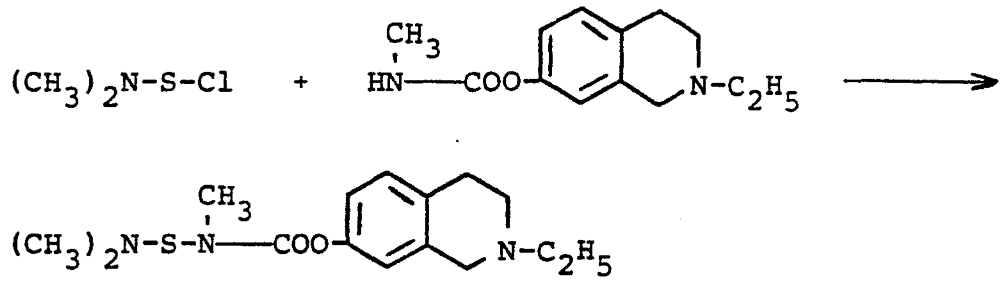

Die Verbindungen der Formel (V) sind bekannt.

Die Reaktion erfolgt in einem inerten Verdünnungsmittel, bevorzugt in Gegenwart von Säurebindemittel und in Gegenwart von Katalysatoren bei Temperaturen von 0-100°C, bevorzugt 50-80°C.

Als Katalysatoren seien genannt z.B. quartäre Ammoniumsalze wie Benzyldimethylammoniumchlorid, ferner Triethylamin-Schwefeldioxid-Komplexe.

Als Verdünnungsmittel seien genannt gegebenenfalls chlorierte Kohlenwasserstoffe z.B. Toluol, Ether z.B. Diethylether.

Zur Entfernung des bei der Reaktion freiwerdenden Chlorwasserstoffs wird entweder bei erhöhter Temperatur gearbeitet oder ein Säurebindemittel wie Alkali- oder Erdalkali-hydroxide, -carbonate, -bicarbonate, -alkoholate oder Amine zugegeben.

Man arbeitet üblicherweise bei Normaldruck. Die Ausgangsprodukte werden üblicherweise im molaren Verhältnis zueinander eingesetzt.

Die Wirkstoffe eignen sich bei guter Pflanzenverträglichkeit und günstiger Warmblütertoxizität zur Bekämpfung von tierischen Schädlingen, insbesondere Insekten, Spinnentieren und Nematoden, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.
Aus der Ordnung der Diplopoda z.B. Blaniulus guttulatus.
Aus der Ordnung der Chilopoda z.B. Geophilus carpophagus, Scutigera spec.
Aus der Ordnung der Symphyla z.B. Scutigerella immaculata.
Aus der Ordnung der Thysanura z.B. Lepisma saccharina.
Aus der Ordnung der Collembola z.B. Onychiurus armatus.
Aus der Ordnung der Orthoptera z.B. Blatta orientalis, Periplaneta americana, Leucophaea maderae, Blattella germanica, Acheta domesticus, Gryllotalpa spp., Locusta

migratoria migratorioides, Melanoplus differentialis, Schistocerca gregaria.
Aus der Ordnung der Dermaptera z.B. Forficula auricularia.
Aus der Ordnung der Isoptera z.B. Reticulitermes spp..
Aus der Ordnung der Anoplura z.B. Phylloxera vastatrix, Pemphigus spp., Pediculus humanus corporis, Haematopinus spp., Linognathus spp.
Aus der Ordnung der Mallophaga z.B. Trichodectes spp., Damalinea spp.

Aus der Ordnung der Thysanoptera z.B. Hercinothrips femoralis, Thrips tabaci.
Aus der Ordnung der Heteroptera z.B. Eurygaster spp., Dysdercus intermedius, Piesma quadrata, Cimex lectularius, Rhodnius prolixus, Triatoma spp.
Aus der Ordnung der Homoptera z.B. Aleurodes brassicae, Bemisia tabaci, Trialeurodes vaporariorum, Aphis gossypii, Brevicoryne brassicae, Cryptomyzus ribis, Doralis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.
Aus der Ordnung der Lepidoptera z.B. Pectinophora gossypiella, Bupalus piniarius, Cheimatobia brumata, Lithocolletis blancardella, Hyponomeuta padella, Plutella

maculipennis, Malacosoma neustria, Euproctis chrysorrhoea, Lymantria spp. Bucculatrix thurberiella, Phyllocnistis citrella, Agrotis spp., Euxoa spp., Feltia spp., Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.
Aus der Ordnung der Hymenoptera z.B. Diprion spp., Hoplocampa spp., Lasius spp., Monomorium pharaonis, Vespa spp.
Aus der Ordnung der Diptera z.B. Aedes spp., Anopheles

spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp..
Aus der Ordnung der Arachnida z.B. Scorpio maurus, Latrodectus mactans.

Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp..

Zu den pflanzenparasitären Nematoden gehören Pratylenchus spp., Radopholus similis, Ditylenchus dipsaci, Tylenchulus semipenetrans, Heterodera spp., Meloidogyne spp., Aphelenchoides spp., Longidorus spp., Xiphinema spp., Trichodorus spp..

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe sowie Butan, Propan, Stickstoff

und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additve können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in ihren handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a.

Die erfindungsgemäßen Wirkstoffe können ferner in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwen-

dungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepaßten üblichen Weise.

Bei der Anwendung gegen Hygiene- und Vorratsschädlinge zeichnen sich die Wirkstoffe durch eine hervorragende Residualwirkung auf Holz und Ton sowie durch eine gute Alkalistabilität auf gekälkten Unterlagen aus.

Herstellungsbeispiele

1. N-Methyl-O-(2-methyl-1,2,3,4-tetrahydro-isochinolin-5-yl)-carbamidsäureester

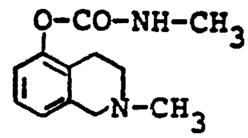

8,1 g (0,05 mol) 5-Hydroxy-2-methyl-1,2,3,4-tetrahydro-isochinolin werden in 50 ml Aceton mit einer Spatelspitze Diazabicyclooctan und 5,7 g (0,10 mol) Methylisocyanat drei Stunden zum Sieden erhitzt. Nach dem Abkühlen kristallisiert die Verbindung durch Zusatz von Diethylether aus. 7,7 g (70 % d.Th.) Schmp. 87-88°C.

2. N-Methyl-O-(2-methyl-1,2,3,4-tetrahydro-isochinolin-7-yl)-carbamidsäureester

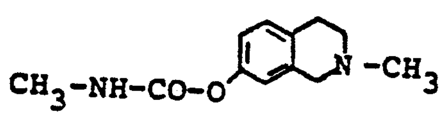

Nach der bei Beispiel 1 angegebenen Arbeitsweise erhält man bei gleicher Ansatzgröße 8,4 g (76 % d.Th.) Produkt mit Schmp. 109-110°C.

3. N-Methyl-O-/2̄-(2-propenyl)-1,2,3,4-tetrahydro-isochinolin-5-yl)-carbamidsäureester

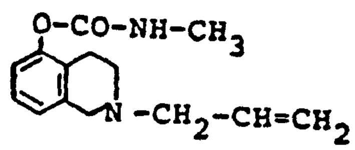

Nach der bei Beispiel 1 angegebenen Arbeitsweise erhält man bei gleicher Ansatzgröße 7,9 g (64 % d.Th.) Produkt mit Schmp. 89-91°C.

4. N-(Dichlorfluormethylthio)-N-methyl-O-(2-methyl-1,2,3,4-tetrahydro-isochinolin-5-yl)-carbamidsäureester

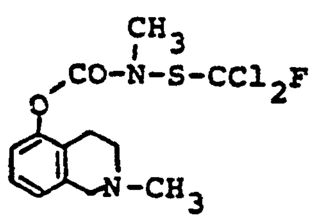

4,9 g (0,03 mol) 5-Hydroxy-2-methyl-1,2,3,4-isochinolin werden in 1 ltr. absolutem Toluol in der Hitze gelöst und dann bei 50°C zunächst mit 6,3 g (0,03 mol) N-(Dichlorfluormethylthio)-N-methylcarbamidsäurefluorid und anschließend mit 3 g (0,03 mol) Triethylamin tropfenweise versetzt. Nach 8-stündigem Nachrühren wird der Niederschlag (5-Hydroxy-2-methyl-1,2,3,4-isochinolin) abfiltriert. Die Mutterlauge wird mit Wasser (3 x 100 ml)

gewaschen, über Calciumchlorid getrocknet, eingeengt und schließlich über Silicagel mit Methanol chromatographiert.
27 g (25 % d.Th.) $R_f$-Wert 0,49 ($SiO_2$, Methanol), IR: 1739 $cm^{-1}$.
Bei der gleichen Arbeitsweise in 100 ml Tetrahydrofuran als Lösungsmittel erhält man das Produkt in einer Ausbeute von 81 %.

5. N-(Dichlorfluormethylthio)-N-methyl-O-(2-methyl-1,2,3,4-tetrahydro-isochinolin-7-yl)-carbamidsäureester

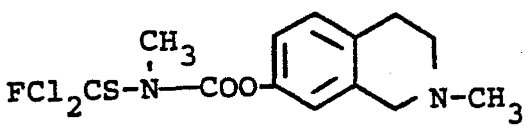

Analog zum vorstehend beschriebenen Beispiel mit Toluol als Lösungsmittel erhält man das Produkt in 40 %iger Ausbeute. $R_f$-Wert 0,43 ($SiO_2$, Methanol), IR: 1744 $cm^{-1}$.

6. N-(Dichlorfluormethylthio)-N-methyl-O-/2-(2-propenyl)-1,2,3,4-tetrahydro-isochinolin-5-yl/-carbamidsäureester

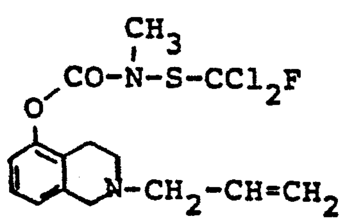

Analog der Verfahrensweise von Beispiel 4 mit Tetrahydrofuran als Lösungsmittel erhält man das Produkt in 77 % Ausbeute. $R_f$-Wert 0,48 ($SiO_2$, Methanol), IR: 1742 $cm^{-1}$.

7. N-(Dibutylaminothio)-N-methyl-O-(2-methyl-1,2,3,4-tetrahydro-isochinolin-5-yl)-carbamidsäureester

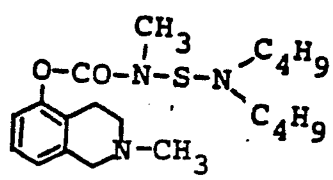

4,1 g (0,025 mol) 5-Hydroxy-2-methyl-1,2,3,4-tetrahydro-isochinolin werden in 15 ml absolutem Dimethylformamid gelöst und portionsweise mit 0,8 g (0,027 mol) Natriumhydrid versetzt. Dann werden 5,9 g (0,025 mol) N-(Dibutylamino-thio)-N-methyl-carbamidsäurefluorid so zugetropft, daß die Reaktionstemperatur 30°C nicht übersteigt. Nach 6 Stunden wird das Reaktionsgemisch mit Wasser versetzt und mit Methylenchlorid extrahiert. Die organische Phase wird über Natriumsulfat getrocknet, eingeengt und über 200 g Silicagel mit Methanol chromatographiert. 3,8 g (40 % d. Th.). $R_f$-Wert 0,57 ($SiO_2$, Essigester) IR: 1710 $cm^{-1}$.

Analog erhält man

8 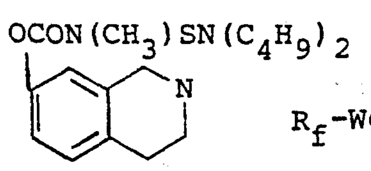 $R_f$-Wert: 0,41 ($SiO_2$,Methanol)

9 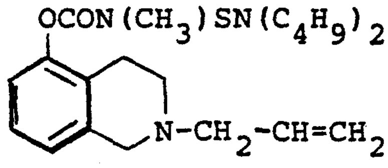

$R_f$-Wert: 0,52 ($SiO_2$, Essigester)

Das Ausgangsmaterial 5-Hydroxy-2-(2-propenyl)-1,2,3,4-tetrahydro-isochinolin kann auf folgende Weise hergestellt werden:

a) 5-Hydroxy-2-(2-propenyl)-isochinoliniumbromid

72 g (0,5 mol) 5-Hydroxy-isochinolin werden in 1000 ml Ethanol bei 40°C mit 121 g (1,0 mol) 3-Brompropen tropfenweise versetzt. Dann wird das Reaktionsgemisch 5 Stunden zum Sieden erhitzt. Beim Abkühlen kristallisieren 75 g mit Schmp. 207-209°C aus. Durch Einengen der Mutterlauge erhält man weitere 24 g Produkt (Schmp. 206-208°C). Die Gesamtausbeute beträgt 74 %.

b) 5-Hydroxy-2-(2-propenyl)-1,2,3,4-tetrahydro-isochinolin

98 g (0,368 mol) 5-Hydroxy-2-(2-propenyl)-isochinoliniumbromid werden in 230 ml Methanol mit 23 ml Wasser vorgelegt und unter Kühlung auf 20 - 25°C mit 53 g (1,39 mol) Natriumborhydrid, gelöst in 210 ml $H_2O$ mit 1 ml 10 %iger Natronlauge, tropfenweise versetzt. Das Edukt geht in

Lösung und bald danach erscheint ein orangefarbener Niederschlag, der sich langsam entfärbt. Man rührt insgesamt 3 Stunden nach und gießt schließlich die Suspension in 3 ltr. Wasser ein. Der Niederschlag wird abgesaugt und aus Toluol umkristallisiert.
51,6 g (74 % d.Th.), Schmp. 130-132°C.

Beispiel A

Phaedon-Larven-Test

Lösungsmittel: 3 Gewichtsteile Dimethylformamid
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Meerrettichblattkäfer-Larven (Phaedon cochleariae) besetzt, solange die Blätter noch feucht sind.

Nach der gewünschten Zeit wird die Abtötung in % bestimmt. Dabei bedeutet 100 %, daß alle Käfer-Larven abgetötet wurden; 0 % bedeutet, daß keine Käfer-Larven abgetötet wurden.

Bei diesem Test zeigen z.B. die folgenden Verbindungen der Herstellungsbeispiele überlegene Wirksamkeit gegenüber dem Stand der Technik: 1, 2, 7, 8, 4, 5, 3, 6, 9

Beispiel B

Myzus-Test

Lösungsmittel: 3 Gewichtsteile Dimethylformamid
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea), die stark von der Pfirsichblattlaus (Myzus persicae) befallen sind, werden durch Tauchen in die Wirkstoffzubereitung der gewünschten Konzentration behandelt.

Nach der gewünschten Zeit wird die Abtötung in % bestimmt. Dabei bedeutet 100 %, daß alle Blattläuse abgetötet wurden; 0 % bedeutet, daß keine Blattläuse abgetötet wurden.

Bei diesem Test zeigen z.B. die folgenden Verbindungen der Herstellungsbeispiele überlegene Wirksamkeit gegenüber dem Stand der Technik: 1, 2, 7, 4, 3, 6, 9

0135084

Beispiel C

Tetranychus-Test (resistent)

Lösungsmittel: 3 Gewichtsteile Dimethylformamid
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Bohnenpflanzen (Phaseolus vulgaris), die stark von allen Entwicklungsstadien der gemeinen Spinnmilbe oder Bohnenspinnmilbe (Tetranychus urticae) befallen sind, werden durch Tauchen in die Wirkstoffzubereitung der gewünschten Konzentration behandelt.

Nach der gewünschten Zeit wird die Abtötung in % bestimmt. Dabei bedeutet 100 %, daß alle Spinnmilben abgetötet wurden; 0 % bedeutet, daß keine Spinnmilben abgetötet wurden.

Bei diesem Test zeigen z.B. die folgenden Verbindungen der Herstellungsbeispiele überlegene Wirksamkeit gegenüber dem Stand der Technik: 1, 2, 7, 8, 4, 5, 3, 6, 9

Beispiel D

Grenzkonzentrations-Test / Wurzelsystemische Wirkung

Testinsekt: Phaedon cochleariae
Lösungsmittel: 3 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit Boden vermischt. Dabei spielt die Konzentration des Wirkstoffs in der Zubereitung praktisch keine Rolle, entscheidend ist allein die Wirkstoffgewichtsmenge pro Volumeneinheit Boden, welche in ppm (= mg/l) angegeben wird. Man füllt den behandelten Boden in Töpfe und bepflanzt diese mit Kohl (Brassica oleracea). Der Wirkstoff kann so von den Pflanzenwurzeln aus dem Boden aufgenommen und in die Blätter transportiert werden.

Für den Nachweis des wurzelsystemischen Effektes werden nach 7 Tagen ausschließlich die Blätter mit den obengenannten Testtieren besetzt. Nach weiteren 2 Tagen erfolgt die Auswertung durch Zählen oder Schätzen der toten Tiere. Aus den Abtötungszahlen wird die wurzelsystemische Wirkung des Wirkstoffs abgeleitet. Sie ist 100 %, wenn alle

Testtiere abgetötet sind und 0 %, wenn noch genau so viele Testinsekten leben wie bei der unbehandelten Kontrolle.

Bei diesem Test zeigen z.B. die folgenden Verbindungen der Herstellungsbeispiele überlegene Wirkung gegenüber dem Stand der Technik: 1, 2, 9, 6, 3

Beispiel E

Grenzkonzentrations-Test / Bodeninsekten

Testinsekt: Phorbia antiqua (im Boden)
Lösungsmittel: 3 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit dem Boden vermischt. Dabei spielt die Konzentration des Wirkstoffs in der Zubereitung praktisch keine Rolle, entscheidend ist allein die Wirkstoffgewichtsmenge pro Volumeneinheit Boden, welche in ppm (= mg/l) angegeben wird. Man füllt den Boden in Töpfe und läßt diese bei Raumtemperatur stehen.

Nach 24 Stunden werden die Testtiere in den behandelten Boden gegeben und nach weiteren 2 bis 7 Tagen wird der Wirkungsgrad des Wirkstoffs durch Auszählen der toten und lebenden Testinsekten in % bestimmt. Der Wirkungsgrad ist 100 %, wenn alle Testinsekten abgetötet worden sind, er ist 0 %, wenn noch genau so viele Testinsekten leben wie bei der unbehandelten Kontrolle.

Bei diesem Test zeigen z.B. die folgenden Verbindungen der Herstellungsbeispiele überlegene Wirkung gegenüber dem Stand der Technik: 7, 8

Beispiel F

Grenzkonzentrations-Test / Nematoden

Testnematode: Meloidogyne incognita
Lösungsmittel: 3 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit Boden vermischt, der mit den Testnematoden stark verseucht ist. Dabei spielt die Konzentration des Wirkstoffs in der Zubereitung praktisch keine Rolle, entscheidend ist allein die Wirkstoffmenge pro Volumeneinheit Boden, welche in ppm angegeben wird. Man füllt den behandelten Boden in Töpfe, sät Salat ein und hält die Töpfe bei einer Gewächshaus-Temperatur von 27°C.

Nach vier Wochen werden die Salatwurzeln auf Nematodenbefall (Wurzelgallen) untersucht und der Wirkungsgrad des Wirkstoffs in % bestimmt. Der Wirkungsgrad ist 100 %, wenn der Befall vollständig vermieden wird, er ist 0 %, wenn der Befall genau so hoch ist wie bei den Kontrollpflanzen in unbehandeltem, aber in gleicher Weise verseuchtem Boden.

Bei diesem Test zeigen z.B. die folgenden Verbindungen der Herstellungsbeispiele überlegene Wirksamkeit gegenüber dem Stand der Technik: 1, 9

Patentansprüche

1. Tetrahydroisochinolinyl-carbamidsäureester der Formel (I)

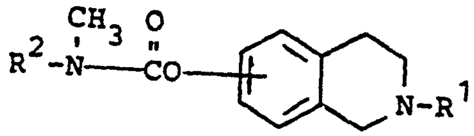

(I)

in welcher

$R^1$ für gegebenenfalls substituiertes Alkyl, Alkenyl oder Alkinyl steht,

$R^2$ für Wasserstoff, Alkyl oder den Rest $R^3$-S-steht, wobei $R^3$ für gegebenenfalls halogensubstituiertes Alkyl, gegebenenfalls substituiertes Dialkylamino steht, wobei die beiden Alkylreste am Stickstoffatom gegebenenfalls über ein Heteroatom (Sauerstoff, gegebenenfalls alkylsubstituierter Stickstoff oder Schwefel) einen Ring bilden können, oder für acyliertes Alkylamino steht.

2. Verfahren zur Herstellung der Tetrahydroisochinolinyl-carbamidsäureester der Formel (I)

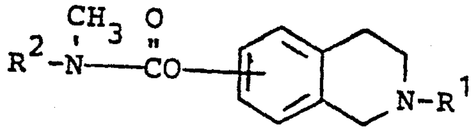

(I)

in welcher

$R^1$ für gegebenenfalls substituiertes Alkyl, Alkenyl oder Alkinyl steht

$R^2$ für Wasserstoff, Alkyl oder den Rest $R^3$-S- steht, wobei $R^3$ für gegebenenfalls halogensubstituiertes Alkyl, gegebenenfalls substituiertes Dialkylamino steht, wobei die beiden Alkylreste am Stickstoffatom gegebenenfalls über ein Heteroatom (Sauerstoff, gegebenenfalls alkylsubstituierter Stickstoff oder Schwefel) einen Ring bilden können, oder für acyliertes Alkylamino steht,

indem man Hydroxy-tetrahydroisochinoline der Formel (II)

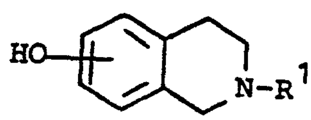

(II)

in welcher

$R^1$ die oben angegebene Bedeutung hat

a) in üblicher Weise mit Methylisocyanat umsetzt oder

b) mit Carbamidsäurehalogeniden der Formel (III)

$$R^2\text{-}\overset{CH_3}{\overset{|}{N}}\text{-}CO\ X \qquad \text{(III)}$$

in welcher

$R^2$ die oben angegebene Bedeutung hat und

X für Halogen steht

gegebenenfalls in Gegenwart eines Säurebindemittels umsetzt, oder

c) Verbindungen der Formel (IV)

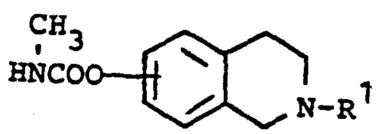

(IV)

in welcher

$R^1$ die oben angegebene Bedeutung hat,

mit Sulfensäurechloriden der Formel (V)

$$R^3\text{-}S\text{-}Cl \qquad \text{(V)}$$

in welcher

$R^2$ die oben angegebene Bedeutung hat,

gegebenenfalls in Gegenwart eines Säurebindemittels, gegebenenfalls in Gegenwart von Katalysatoren umsetzt.

3. Verbindungen der Formel (I) gemäß Anspruch 1, in welcher

$R^1$ für gegebenenfalls substituiertes Alkyl mit 1 bis 6 C-Atomen, für gegebenenfalls substituiertes Alkenyl mit 3 bis 6 C-Atomen oder für gegebenenfalls substituiertes Alkinyl mit 3 bis 6 C-Atomen und

$R^2$ für Wasserstoff, Alkyl mit 1 bis 4 C-Atomen, sowie den Rest $-S-R^3$ steht, $R^3$ steht für gegebenenfalls halogeniertes Alkyl mit 1-3 C-Atomen, gegebenenfalls substituiertes Dialkyl wobei die beiden Alkylreste am Stickstoffatom auch gegebenenfalls über ein Heteroatom (Sauerstoff, gegebenenfalls durch $C_{1-4}$ substituierten Stickstoff, Schwefel) einen Ring bilden können, mit insgesamt 2 bis 16 C-Atomen stehen, oder für $C_{1-4}$-Alkoxycarbonyl-$C_{1-4}$-alkylamino, wobei

als Substituenten Halogen, Cyan, Nitro, Alkyl mit 1-2 C-Atomen, Alkyloxycarbonyl mit 1-5 C-Atomen und Trifluormethyl genannt seien und der Carbamidsäurerest in 5- oder 7-Stellung steht.

4. Verbindungen der Formel (I) gemäß Anspruch 1, in welcher

$R^1$ für Methyl, Ethyl, Propyl, Butyl, Allyl oder Propargyl steht und

$R^2$ für Wasserstoff, Methyl, oder $-S-R^3$ steht, $R^3$ steht für Trichlormethyl, Fluordichlormethyl, Dimethylamino, Diethylamino, Dipropylamino, Dibutylamino, Dipentylamino, Di(ethoxycarbonylmethyl)amino, Pyrrolidino, Piperidino, Morpholino, N'-Methyl-piperazino, Methoxycarbonyl-butylamino,

und in denen der Carbamidsäurerest in 5- oder 7-Stellung steht.

5. Schädlingsbekämpfungsmittel, gekennzeichnet durch einen Gehalt an mindestens einem Tetrahydroisochinolinylcarbamidsäureester der Formel (I) gemäß Anspruch 1.

6. Verwendung von Tetrahydroisochinolinylcarbamidsäureester der Formel (I) gemäß Anspruch 1 zur Bekämpfung von Schädlingen.

7. Verfahren zur Bekämpfung von Schädlingen, dadurch gekennzeichnet, daß man Tetrahydroisochinolinylcarbamidsäureester der Formel (I) gemäß Anspruch 1 auf Schädlinge und/oder ihren Lebensraum einwirken läßt.

8. Verfahren zur Herstellung von Schädlingsbekämpfungsmitteln, dadurch gekennzeichnet, daß man Tetrahydroisochinolinylcarbamidsäureester der Formel (I) gemäß Anspruch 1 mit Streckmitteln und/oder oberflächenaktiven Mitteln vermischt.